(19) Europäisches Patentamt
European Patent Office
Office européen des brevets

(11) Veröffentlichungsnummer: **0 057 888**
**B1**

# (12) EUROPÄISCHE PATENTSCHRIFT

(45) Veröffentlichungstag der Patentschrift: **02.10.85**

(51) Int. Cl.[4]: **C 07 C 127/22,** A 01 N 47/34

(21) Anmeldenummer: **82100679.8**

(22) Anmeldetag: **01.02.82**

(54) **N-Benzoyl-N'-phenoxyphenylharnstoffe, Verfahren zu ihrer Herstellung und ihre Verwendung zur Bekämpfung von Schädlingen.**

(30) Priorität: **07.02.81 DE 3104407**

(43) Veröffentlichungstag der Anmeldung: **18.08.82 Patentblatt 82/33**

(45) Bekanntmachung des Hinweises auf die Patenterteilung: **02.10.85 Patentblatt 85/40**

(84) Benannte Vertragsstaaten: **AT BE CH DE FR GB IT LI LU NL SE**

(56) Entgegenhaltungen:
**EP - A - 0 044 410**
**DE - A - 2 531 202**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch-Strasse 38, D-6700 Ludwigshafen (DE)**

(72) Erfinder: **Lange, Arno, Dr., Friedrichsplatz 11, D-6800 Mannheim (DE)**
Erfinder: **Kiehs, Karl, Dr., Sudetenstrasse 22, D-6840 Lampertheim (DE)**
Erfinder: **Adolphi, Heinrich, Dr., Kalmitweg 11, D-6703 Limburgerhof (DE)**
Erfinder: **Becker, Rainer, Dr., Sonnenwendstrasse 83, D-6702 Bad Duerkheim (DE)**

**EP 0 057 888 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft N-Benzoyl-N'-phenoxyphenylharnstoffe, Verfahren zu ihrer Herstellung und Schädlingsbekämpfungsmittel, die diese Verbindungen als Wirkstoffe enthalten.

Es ist bereits bekannt, daß N-Benzoyl-N-phenylharnstoffe als Insektizide verwendet werden können (J. Agr. Food Chem. 21, 348 (1973); DE-OS 25 31 202).

Es wurde nun gefunden, daß N-Benzoyl-N'-phenoxyphenylharnstoffe der Formel

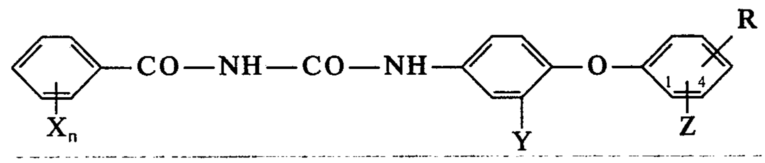

(I)

in der

Z Chlor in 3-Stellung oder Methyl,
R Fluor, Chlor, Brom, einen gegebenenfalls substituierten Phenoxyrest, einen gegebenenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Y Wasserstoff, Fluor, Chlor oder Brom,
X Fluor, Chlor, Brom, Methyl oder Nitro und
n 2 oder 3 bedeuten, wobei die Substituenten X gleich oder verschieden sind,

in der

Z Wasserstoff bedeutet, wenn
R einen gegebenenfalls durch Fluor, Chlor, Brom, Halogenalkyl- oder Halogenalkoxyreste mit bis zu 4 Kohlenstoffatomen substituierten Phenoxyrest, eine gegebenenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Y Wasserstoff, Fluor oder Chlor,
X Fluor, Chlor, Brom, Methyl oder Nitro und
n 1, 2 oder 3 bedeuten, wobei die Substituenten X gleich oder verschieden sind,

in der

Z Wasserstoff bedeutet, wenn
R Fluor oder Brom,
Y Chlor
X Fluor, Chlor, Brom, Methyl oder Nitro und
n 1 bedeuten,

in der

Y Brom oder Nitro bedeutet, wenn
R Fluor, Chlor, Brom, einen gegebenenfalls durch Fluor, Chlor, Brom, Halogenalkyl- oder Halogenalkoxyreste mit bis zu 4 Kohlenstoffatomen substituierten Phenoxyrest, einen gegebenenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Z Wasserstoff, Chlor in m-Stellung oder Methyl,
X Fluor, Chlor, Brom, Methyl oder Nitro und
n 1, 2 oder 3 bedeuten, wobei die Substituenten X gleich oder verschieden sind,

Schädlinge, insbesondere Insekten, wirksamer bekämpfen als bekannte N-Benzoyl-N'-phenoxyphenylharnstoffe.

Gegebenenfalls substituierte Phenoxyreste für R in Formel I sind beispielsweise durch Fluor, Chlor, Brom, Halogenalkyl- oder Halogenalkoxyreste mit bis zu 4 Kohlenstoffatomen substituierte Phenoxyreste.

Als Alkoxyreste, die durch Chlor und/oder Fluor substituiert sein können, kommen für R z. B. Methoxy, Ethoxy, n-Propoxy, i-Propoxy, Trifluormethoxy, 1,1,2,2-Tetrafluorethoxy, Difluormethoxy, 2-Chlor-2,1,1-trifluorethoxy, 1,1,2,3,3,3-Hexafluorpropoxy, 2-Brom-2,1,1-trifluorethoxy, als Alkylreste

Methyl, Ethyl, n-Propyl und Isopropyl, vorzugsweise Methyl, und als 2-Hydroxyhexahalogenisopropylreste der 2-Hydroxyhexafluorisopropylrest, der 2-Hydroxy-dichlortetrafluorisopropylrest oder der 2-Hydroxytrichlortrifluorisopropylrest in Betracht.

Bevorzugte Verbindungen der Formel 1 sind solche, bei denen Z Wasserstoff, X Chlor in o-Stellung und n 1 oder solche, bei denen X Chlor oder Fluor in o-Stellung und n 2 bedeuten.

Man erhält die N-Benzoyl-N'-phenoxyphenylharnstoffe durch Umsetzung von

a) einer Verbindung der Formel

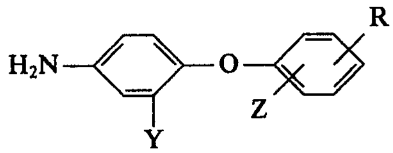

(II)

in der R, Y und Z die obengenannten Bedeutungen haben, mit einem Benzoylisocyanat der Formel

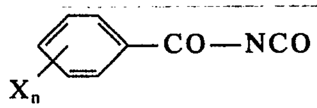

(III)

in der X und n die obengenannten Bedeutungen haben, oder

b) durch Umsetzung einer Verbindung der Formel

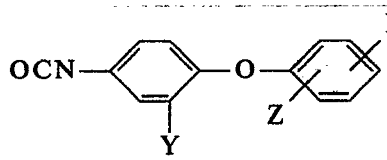

(IV)

in der Y, Z und R die obengenannten Bedeutungen haben, mit einem Benzamid der Formel

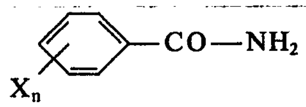

(V)

in der X und n die obengenannten Bedeutungen haben.

Beide Umsetzungen werden vorzugsweise in Lösungs- bzw. Verdünnungsmittel durchgeführt. Als solche kommen praktisch alle inerten organischen Solventien in Betracht. Hierzu gehören insbesondere aliphatische und aromatische, gegebenenfalls chlorierte oder nitrierte Kohlenwasserstoffe, wie Benzol, Toluol, Xylole, Chlorbenzole, Benzin, Tetrachlorkohlenstoff, 1,2-Dichlorethan, Methylenchlorid, Chloroform, Nitromethan, cyclische und acyclische Ether, wie Diethylether, Tetrahydrofuran, Dioxan, acyclische und cyclische Ketone, wie Aceton, Methylethylketon, Methylisopropylketon, Cyclohexanon, außerdem Nitrile, wie Acetonitril, Benzonitril. Auch Gemische dieser Lösungsmittel können verwendet werden.

Bei der Umsetzung a) kann die Reaktionstemperatur innerhalb eines größeren Bereiches variiert werden. Sie liegt in der Regel zwischen 0 und 80°C, vorzugsweise zwischen 20 und 60°C. In diesem bevorzugten Bereich verläuft die Umsetzung ohne zusätzliche Kühlung. Bei der Umsetzung b) kann die Temperatur zwischen 20 und 160°C variiert werden, vorzugsweise arbeitet man bei einer Temperatur zwischen 80 und 130°C.

Man läßt die Umsetzung im allgemeinen bei Normaldruck ablaufen.

Die Reaktionskomponenten werden vorzugsweise in äquimolarem Verhältnis eingesetzt. Ein Überschuß der einen oder anderen Komponente bringt keine wesentlichen Vorteile. Die Umsetzung verläuft praktisch quantitativ.

Die erfindungsgemäßen Verbindungen fallen in der Regel analysenrein an; andernfalls können sie durch Umkristallisieren gereinigt werden. Zu ihrer Charakterisierung dienen Elementaranalyse und Schmelzpunkt.

Zur Durchführung der Verfahrensvariante a) wird zweckmäßigerweise das substituierte Anilin der Formel II zusammen mit dem Lösungs- oder Verdünnungsmittel vorgelegt, dann wird eine äquimolare Menge an Isocyanat der Formel III zugegeben. Nach mehrstündiger Reaktionsdauer, in der Regel nach zwei Stunden, wird das Produkt dann abgesaugt oder die Reaktionslösung wird am Rotationsverdampfer vom Lösungsmittel befreit. Anschließend wird das Produkt unter vermindertem Druck getrocknet. Bei der Verfahrensvariante b) beträgt die Reaktionsdauer 2 bis 6 Stunden.

Die Phenoxyaniline der Formel II und die Benzoylisocyanate der Formel III lassen sich nach bekannten Methoden herstellen (J. Org. Chem. 28, 1805 bis 1811 (1963); Weygand-Hilgetag, Organisch-Chemische Experimentierkunst, 4. Auflage, J. A. Barth-Verlag, Leipzig, 1970, S. 374, 510 ff). Die Aminogruppe der Verbindungen der Formel II kann nach üblichen Verfahren, z. B. durch Umsetzung mit Phosgen, in die Isocyanatgruppe umgewandelt werden, wobei die Verbindungen der Formel IV erhalten werden.

## Herstellungsbeispiel

7,1 g 3-Brom-4-(4-fluorphenoxy)-anilin werden bei Raumtemperatur in 50 ml Toluol gelöst. Dann werden 4,9 g 2,6-Difluorbenzoylisocyanat zugetropft, wobei die Temperatur um 15°C steigt. Danach rührt man eine Stunde bei 50°C, läßt abkühlen und saugt den Niederschlag ab. Man erhält 8,5 g N-(2,6-Difluorbenzoyl)-N'-[4-(4-fluorphenoxy)-3-brom-phenyl]-harnstoff;
Fp = 162 bis 164°C (Wirkstoff Nr. 1).

Folgende Verbindungen lassen sich beispielsweise analog oder nach einem der oben beschriebenen Verfahren herstellen:

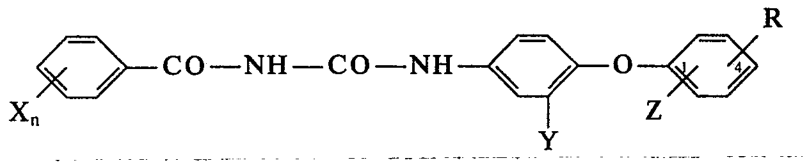

| Nr. | $X_n$ | Y | Z | R | Fp [°C] |
|---|---|---|---|---|---|
| 2 | 2-F, 6-F | Cl | H | 4-$OCHF_2$ | 148—153 |
| 3 | 2-F, 6-F | Cl | H | 4-$OCF_2CHClF$ | 156—159 |
| 4 | 2-Cl, 6-Cl | Cl | H | 4-$OCHF_2$ | 171—174 |
| 5 | 2-Cl, 6-Cl | Cl | H | 4-$OCF_2CHClF$ | 129—139 |
| 6 | 2-$NO_2$ | Cl | H | 4-$OCHF_2$ | 192—194 |
| 7 | 2-$NO_2$ | Cl | H | 4-$OCF_2CHClF$ | 185—188 |
| 8 | 2-$CH_3$ | Cl | H | 4-$OCHF_2$ | 160—162 |
| 9 | 2-Cl | Cl | H | 4-$OCF_2CHClF$ | 128—130 |
| 10 | 2-F, 6-F | Cl | 3-$CH_3$ | 4-Cl | 205—206 |
| 11 | 2-Cl, 6-Cl | Cl | 3-$CH_3$ | 4-Cl | 205—210 |
| 12 | 2-F, 6-F | Cl | 2-$CH_3$ | 4-Cl | 200—202 |
| 13 | 2-Cl, 6-Cl | Cl | 3-Cl | 4-Cl | 240—242 |
| 14 | 2-F, 6-F | Cl | 3-Cl | 4-Cl | 217—220 |
| 15 | 2-F, 6-F | Cl | H | 4-$C(OH)(CF_3)_2$ | 170—185 |
| 16 | 2-Cl | Cl | H | 4-$C(OH)(CF_3)_2$ | 165—177 |
| 17 | 2-Cl | H | H | 4-$OCF_2$-$CHClF_2$ | 169—173 |
| 18 | 2-F, 6-F | H | H | 4-$OCF_2$-$CHClF_2$ | 184—186 |
| 19 | 2-F, 6-F | H | H | 4-$OC_6H_5$ | 164—210 |
| 20 | 2-Cl | H | H | 4-$OC_6H_5$ | 166—203 |
| 21 | 2-Cl | Cl | H | 4-Br | 155—156 |

(Fortsetzung)

| Nr. | $X_n$ | Y | Z | R | Fp [°C] |
|---|---|---|---|---|---|
| 22 | 2-Cl | Br | H | 4-F | 162—164 |
| 23 | 2-Cl, 6-Cl | F | H | 4-$CH_3$ | 212—217 |
| 24 | 2-Cl | F | H | 4-$CH_3$ | 168—173 |
| 25 | 2-F, 6-F | F | H | 4-$CH_3$ | 195—200 |
| 26 | 2-F, 6-F | $NO_2$ | H | 4-Cl | 196—200 |
| 27 | 2-Cl | $NO_2$ | H | 4-Cl | 186—191 |
| 28 | 2-F, 6-F | H | H | 4-$OCHF_2$ | 214—216 |
| 29 | 2-Cl, 6-Cl | H | H | 4-$OCHF_2$ | |
| 30 | 2-Cl | H | H | 4-$OCHF_2$ | |
| 31 | 2-F, 6-F | F | H | 4-$OCHF_2$ | |
| 32 | 2-Cl, 6-Cl | F | H | 4-$OCHF_2$ | |
| 33 | 2-Cl | F | H | 4-$OCHF_2$ | |
| 34 | 2-F, 6-F | Br | H | 4-$OCHF_2$ | |
| 35 | 2-Cl, 6-Cl | Br | H | 4-$OCHF_2$ | |
| 36 | 2-Cl | Br | H | 4-$OCHF_2$ | |
| 37 | 2-F, 6-F | F | H | 4-$OCH_2CHClF$ | |
| 38 | 2-Cl, 6-Cl | F | H | 4-$OCF_2CHClF$ | |
| 39 | 2-Cl | F | H | 4-$OCF_2CHClF$ | |
| 40 | 2-F, 6-F | Br | H | 4-$OCF_2CHClF$ | |
| 41 | 2-Cl, 6-Cl | Br | H | 4-$OCF_2CHClF$ | |
| 42 | 2-Cl | Br | H | 4-$OCF_2CHClF$ | |
| 43 | 2-F, 6-F | H | H | 4-$C(OH)(CF_3)_2$ | |
| 44 | 2-Cl, 6-Cl | H | H | 4-$C(OH)(CF_3)_2$ | |
| 45 | 2-Cl | H | H | 4-$C(OH)(CF_3)_2$ | |
| 46 | 2-F, 6-F | F | H | 4-$C(OH)(CF_3)_2$ | |
| 47 | 2-Cl, 6-Cl | F | H | 4-$C(OH)(CF_3)_2$ | |
| 48 | 2-Cl | F | H | 4-$C(OH)(CF_3)_2$ | |
| 49 | 2-F, 6-F | Br | H | 4-$C(OH)(CF_3)_2$ | |
| 50 | 2-Cl, 6-Cl | Br | H | 4-$C(OH)(CF_3)_2$ | |
| 51 | 2-F, 6-F | Cl | H | 4-$OCF_2CHFCF_3$ | 144—146 |

(Fortsetzung)

| Nr. | $X_n$ | Y | Z | R | Fp [°C] |
|---|---|---|---|---|---|
| 52 | 2-F, 6-F | H | 3-Cl | 5-Cl | 220—225 |
| 53 | 2-Cl, 6-Cl | H | 3-Cl | 5-Cl | 231—234 |
| 54 | 2-Cl, 6-F | Cl | H | 4-$OCHF_2$ | 125—133 |
| 55 | 2-Cl, 6-F | Cl | H | 4-$OCH_3$ | 212—220 |
| 56 | 2-Cl | Cl | H | 4-$OCH_3$ | 212—216 |

Die erfindungsgemäßen N-Benzoyl-N'-phenoxyphenylharnstoffe der Formel I sind geeignet, Schädlinge aus der Klasse der Insekten wirksam zu bekämpfen. Sie sind geeignete Adultizide und Ovizide und können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Plutella maculipennis (Kohlschabe), Leucoptera coffeella (Kaffeemotte), Hyponomeuta malinellus (Apfelbaumgespinstmotte), Argyresthia conjugella (Apfelmotte), Sitotroga cerealella (Getreidemotte), Phthorimaea operculella (Kartoffelmotte), Capua reticulana (Apfelschalenwickler), Sparganothis pilleriana (Springwurm), Cacoecia murinana (Tannentriebwickler), Tortrix viridana (Eichenwickler), Clysia ambiguella (Heu- und Sauerwurm), Evetria buoliana (Kieferntriebwickler), Polychrosis botrana (Bekreuzter Traubenwickler), Cydia pomonella (Obstmade), Laspeyresia molesta (Pfirsichtriebbohrer), Laspeyresia funebrana (Pflaumenwickler), Ostrinia nubilalis (Maiszünsler), Loxostege sticticalis (Rübenzünsler), Ephestia kuehniella (Mehlmotte), Chilo suppressalis (Reisstengelbohrer), Galleria Mellonella (Wachsmotte), Malacosoma neustria (Ringelspinner), Dendrolimus pini (Kiefernspinner), Thaumatopoea pityocampa (Pinienprozessionsspinner), Phalera bucephala (Mondfleck), Cheimatobia brumata (Kleiner Frostspanner), Hibernia defoliaria (Großer Frostspanner), Bupalus piniarius (Kiefernspanner), Hyphantria cunea (Weißer Bärenspinner), Agrotis segetum (Wintersaateule), Agrotis ypsilon (Ypsiloneule), Barathra brassicae (Kohleule), Cirphis unipuncta (Heerwurm), Prodenia litura (Baumwollraupe), Laphygma exigua (Rüben-Heerwurm), Panolis flammea (Forleule), Earias insulana (Baumwollkapselwurm), Plusia gamma (Gammaeule), Alabama argillacea (Baumwollblattwurm), Lymantria dispar (Schwammspinner), Lymantria monacha (Nonne), Pieris brassicae (Kohlweißling), Aporia crataegi (Baumweißling);

aus der Ordnung der Käfer (Coleoptera) beispielsweise Blitophaga undata (Schwarzer Rübenaaskäfer), Melanotus communis (Drahtwurm), Limonius californicus (Drahtwurm), Agriotes lineatus (Saatschnellkäfer), Agriotes obscurus (Humusschnellkäfer), Agrilus sinuatus (Birnbaum-Prachtkäfer), Meligethes aeneus (Rapsglanzkäfer), Atomaria linearis (Moosknopfkäfer), Epilachna varivestris (Mexikanischer Bohnenkäfer), Phyllopertha horticola (Junikäfer), Popillia japonica (Japankäfer), Melolontha melolontha (Feldmaikäfer), Melolontha hippocastani (Waldmaikäfer), Amphimallus solstitialis (Brachkäfer), Crioceris asparagi (Spargelhähnchen), Lema melanopus (Getreidehähnchen), Leptinotarsa decemlineata (Kartoffelkäfer), Phaedon cochleariae (Meerrettich-Blattkäfer), Phyllotreta nemorum (Kohlerdfloh), Chaetocnema tibialis (Rübenflohkäfer), Phylloides chrysocephala (Raps-Flohkäfer), Diabrotica 12-punctata (Südlicher Maiswurzelturm), Cassida nebulosa (Nebliger Schildkäfer), Bruchus lentis (Linsenkäfer), Bruchus rufimanus (Pferdebohnenkäfer), Bruchus pisorum (Erbsenkäfer), Sitona lineatus (Linierter Blattrandkäfer), Otiorrhynchus sulcatus (Gefurchter Lappenrüßler), Otiorrhynchus ovatus (Erdbeerwurzelrüßler), Hylobies abietis (Großer Brauner Rüsselkäfer), Byctiscus betulae (Rebenstecher), Anthonomus pomorum (Apfelblütenstecher), Anthonomus grandis (Kapselkäfer), Ceuthorrhynchus assimilis (Kohlschotenrüßler), Ceuthorrhynchus napi (Großer Kohltriebrüßler), Sitophilus granaria (Kornkäfer), Anisandrus dispar (Ungleicher Holzborkenkäfer), Ips typographus (Buchdrukker), Blastophagus piniperda (Gefurchter Waldgärtner);

aus der Ordnung der Zweiflügler (Diptera) beispielsweise Lycoria pectoralis, Mayetiola destructor (Hessenfliege), Dasineura brassicae (Kohlschoten-Gallmücke), Contarinia tritici (Gelbe Weizen-Gallmücke), Haplodiplosis equestris (Sattelmücke), Tipula paludosa (Wiesenschnake), Tipula oleracea (Kohlschnake), Dacus cucurbitae (Melonenfliege), Dacus oleae (Olivenfliege), Ceratitis capitata (Mittelmeerfruchtfliege), Rhagoletis cerasi (Kirschfruchtfliege), Rhagoletis pomonella (Apfelmade), Anastrepha ludens (Mexikanische Fruchtfliege), Oscinella frit (Fritfliege), Phorbia coarctata (Brachfliege), Phorbia antiqua (Zwiebelfliege), Phorbia brassicae (Kleine Kohlfliege), Pegomya hyoscyami (Rübenfliege), Anopheles maculipennis, Culex pipiens, Aedes aegypti (Gelbfiebermücke), Aedes vexans, Tabanus bovinus (Rinderbremse), Tipula paludosa (Wiesenschnake), Musca domestica (Stubenfliege), Fannia canicularis (Kleine Stubenfliege), Muscina stabulans, Glossina morsitans (Tsetse-Fliege),

Oestrus ocis, Chrysomya macellaria, Chrysomya hominivorax, Lucilia cuprina, Lucilia sericata, Hypoderma lineata;
aus der Ordnung der Hautflügler (Hymenoptera) beispielsweise Athalia rosae (Rübenblattwespe), Hoplocampa minuta (Pflaumensägewespe), Monomorium pharaonis (Pharaoameise), Solenopsis geminata (Feuerameise), atta sexdens (Blattschneiderameise);
aus der Ordnung der Wanzen (Heteroptera) beispielsweise Nezara viridula (Grüne Reiswanze), Eurygaster integriceps (Asiatische Getreidewanze), Blissus leucopterus (Chinch bug), Dysdercus cingulatus (Kapok-Wanze), Dysdercus intermedius (Baumwollwanze), Piesma quadrata (Rübenwanze), Lygus pratensis (Gemeine Wiesenwanze);
aus der Ordnung der Pflanzensauger (Homoptera) beispielweise Perkinsiella saccharicida (Zuckerrohrzikade), Nilaparvata lugens (Braune Zikade), Empoasca fabae (Kartoffelzikade), Psylla mali (Apfelblattsauger), Psylla piri (Birnblattsauger), Trialeurodes vaporariorum (Weiße Fliege), Aphis fabae (Schwarze Bohnenlaus), Aphis pomi (Grüne Apfellaus), Aphis sambuci (Holunderblattlaus), Aphidula nastrutii (Kreuzdornblattlaus), Cerosipha gossypii (Gurkenblattlaus), Sappaphis mali (Rosige Apfellaus), Sappaphis mala (Mehlige Birnblattlaus), Dysahpis radicola (Mehlige Apfelfalterlaus), Brachycaudus cardui (Große Pflaumenblattlaus), Brevicoryne brassicae (Kohlblattlaus), Phorodon humuli (Hopfenblattlaus), Rhopalomyzus ascalonicus (Zwiebellaus), Myzodes persicae (Grüne Pfirsichlaus), Myzus cerasi (Schwarze Sauerkirschenlaus), Dysaulacorthum pseudosolani (Gefleckte Kartoffellaus), Acyrthosiphon onobrychis (Grüne Erbsenlaus), Macrosiphon rosae (Große Rosenblattlaus), Megoura viciae (Wickenlaus), Schizoneura lanuginosa (Birnenblattlaus), Pemphigus bursarius (Salatwurzellaus), Dreyfusia nordmannianae (Tannentrieblaus), Dreyfusia piceae (Weißtannenstammlaus), Adelges laricis (Rote Fichtengallenlaus), Viteus vitifolii (Reblaus);
aus der Ordnung der Termiten (Isoptera) beispielsweise Reticulitermes lucifucus, Calotermes flavicollis, Leucotermes flavipes, Termes natalensis;
aus der Ordung der Geradflügler (Orthoptera) beispielsweise Forticula auricularia (Gemeiner Ohrwurm), Acheta domestica (Heimchen), Gryllotalpa gryllotalpa (Maulwurfsgrille), Tachycines asynamorus (Gewächshausschrecke), Locusta migratoria (Wanderheuschrecke), Stauronotus maroccanus (Marokkanische Wanderheuschrecke), Schistocerca peregrina (Wanderheuschrecke), Nomadacris septemfasciata (Wanderheuschrecke), Melanoplus spretus (Felsengebirgsheuschrecke), Melano plus femur-rubrum (Rotbeinige Heuschrecke), Blatta orientalis (Küchenschabe), Blattella germanica (Deutsche Schabe), Periplaneta americana (Amerikanische Schabe), Blabera gigantea (Riesenschabe).

Die Wirkstoffe können als solche, in Form ihrer Formulierungen oder den daraus bereiteten Anwendungsmöglichkeiten, z. B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphtalin, alkylierte Naphtaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphtalinsulfonsäure, Phenolsulfonsäure, Alkylarylsulfonate, Alkylsulfate, Alkylsulfonate, Alkali- und Erdalkalisalze der Dibuthylnaphtalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphtalin und Naphtalinderivaten mit Formaldehyd, Kondensationsprodukte des Naphtalin bzw. der Naphtalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Beispiele für Formulierungen sind:

I. 3 Gewichtsteile der Verbindung Nr. 1 werden mit 97 Gewichtsteilen feinteiligem Kaolin innig vermischt. Man erhält auf diese Weise ein Stäubemittel, das 3 Gew.-% des Wirkstoffs enthält.

II. 30 Gewichtsteile der Verbindung Nr. 10 werden mit einer Mischung aus 92 Gewichtsteilen pulverförmigen Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

III. 10 Gewichtsteile der Verbindung Nr. 11 werden in einer Mischung gelöst, die aus 90 Gewichtsteilen Xylol, 6 Gewichtsteilen des Anlagerungsproduktes von 8 bis 10 Mol Äthylenoxid an 1 Mol Ölsäure-N-monoethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

IV. 20 Gewichtsteile der Verbindung Nr. 18 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

V. 80 Gewichtsteile der Verbindung Nr. 23 werden mit 3 Gewichtsteilen des Natriumsalzes der Diisobutylnaphtalin-alpha-sulfonsäure, 10 Gewichtsteilen des Natriumsalzes einer Ligninsulfonsäure aus einer Sulfit-Ablauge und 7 Gewichtsteilen pulverförmigen Kieselsäuregel gut vermischt und in einer Hammermühle vermahlen.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten im allgemeinen zwischen 0,1 und 95 Gew.-% Wirkstoff, vorzugsweise zwischen 0,5 und 90 Gew.-%.

Die Wirkstoffkonzentrationen in den anwendungsfertigen Zubereitungen können in größeren Bereichen variiert werden. Im allgemeinen liegen sie zwischen 0,0001 und 10%, vorzugsweise zwischen 0,01 und 1%.

Die Wirkstoffe können auch mit gutem Erfolg im Ultra-Low-Volume-Verfahren (ULV) verwendet werden, wobei es möglich ist, Formulierungen mit mehr als 95 Gew.-% Wirkstoff oder sogar den Wirkstoff ohne Zusätze auszubringen.

Die Aufwandmenge an Wirkstoff beträgt unter Freilandbedingungen 0,2 bis 10, vorzugsweise 0,5 bis 2,0 kg/ha.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1 : 10 bis 10 : 1 zugemischt werden.

Beispielsweise können folgende Mittel zugemischt werden:

1,2-Dibrom-3-chlorpropan,
1,3-Dichlorpropen,
1,3-Dichlorpropen + 1,2-Dichlorpropan,
1,2-Dibrom-ethan,
2-sec.-Butyl-phenyl-N-methylcarbamat,
o-Chlorphenyl-N-methylcarbamat,
3-Isopropyl-5-methylphenyl-N-methylcarbamat,
o-Isopropoxyphenyl-N-methylcarbamat,
3,5-Dimethyl-4-methylmercapto-phenyl-N-methylcarbamat,
4-Dimethylamino-3,5-xylyl-N-methylcarbamat,
2-(1,3-Dioxolan-2-yl)-phenyl-N-methylcarbamat,
1-Naphthyl-N-methylcarbamat,
2,3-Dihydro-2,2-dimethyl-benzofuran-7-yl-N-methylcarbamat,
2,2-Dimethyl-1,3-benzodioxol-4-yl-N-methylcarbamat,
2-Dimethylamino-5,6-dimethyl-4-pyrimidinyl-dimethylcarbamat,
2-Methyl-2-(methylthio)-propionaldehyd-O-(methylcarbamoyl)-oxim,
S-Methyl-N-[(methylcarbamoyl)-oxy]-thio-acetimidat,
Methyl-N',N'-dimethyl-N-[(methylcarbamoyl)oxy]-l-thiooxamidat,
N-(2-Methyl-chlor-phenyl)-N',N'-dimethylformamidin,
Tetrachlorthiophen,
1-(2,6-Difluor-benzoyl)-3-(4-chlor-phenyl)-harnstoff,
O,O-Dimethyl-O-(p-nitrophenyl)-phosphorthioat,
O,O-Diethyl-O-(p-nitrophenyl)-phosphorthioat,
O-Ethyl-O-(p-nitrophenyl)-phenyl-phosphonothioat,

O,O-Dimethyl-O-(3-methyl-4-nitrophenyl)-phosphorthioat,
O,O-Diethyl-O-(2,4-dichlorphenyl)-phosphorthioat,
O-Ethyl-O-(2,4-dichlorphenyl)-phenyl-phosphonothioat,
O,O-Dimethyl-O-(2,4,5-trichlorphenyl)-phosphorthioat,
O-Ethyl-O-(2,4,5-trichlorphenyl)-ethyl-phosphonothioat,
O,O-Dimethyl-O-(4-brom-2,5-dichlorphenyl)-phosphorthioat,
O,O-Dimethyl-O-(3-methyl-4-methylthiophenyl)-phosphorthioat,
O-Ethyl-O-(3-methyl-4-methylthiophenyl)-isopropyl-phosphoramidat,
O,O-Diethyl-O-[p-methylsulfinyl-phenyl]-phosphorthioat,
O-Ethyl-S-phenyl-ethyl-phosphonodithioat,
O,O-Diethyl-[2-chlor-1-(2,4-dichlorphenyl)-vinyl]-phosphat,
O,O-Dimethyl-[-2-chlor-1-(2,4,5-trichlorphenyl)]-vinyl-phosphat,
O,O-Dimethyl-S-(1'-phenyl)-ethylacetat-phosphordithioat,
Bis-(dimethylamino)-fluorphosphinoxid,
Octamethyl-pyrophosphoramid,
O,O,O,O-Tetraethyldithio-pyrophosphat,
S-Chlormethyl-O,O-diethyl-phosphordithioat,
O-Ethyl-S,S-dipropyl-phosphordithioat,
O,O-Dimethyl-O-2,2-dichlorvinyl-phosphat,
O,O-Dimethyl-1,2-dibrom-2,2-dichlorethylphosphat,
O,O-Dimethyl-2,2,2-trichlor-1-hydroxy-ethylphosphonat,
O,O-Dimethyl-S-[1,2-biscarbethoxy-ethyl-(1)]-phosphordithioat,
O,O-Dimethyl-O-(1-methyl-2-carbmethoxy-vinyl)-phosphat,
O,O-Dimethyl-S-(N-methyl-carbamoyl-methyl)-phosphordithioat,
O,O-Dimethyl-S-(N-methyl-carbamoyl-methyl)-phosphorthioat,
O,O-Dimethyl-S-(N-methoxyethyl-carbamoyl-methyl)-phosphordithioat,
O,O-Dimethyl-S-(N-formyl-N-methyl-carbamoylmethyl-phosphordithioat,
O,O-Dimethyl-O-[1-methyl-2-(methyl-carbamoyl)-vinyl]-phosphat,
O,O-Dimethyl-O-[(1-methyl-2-dimethylcarbamoyl)-vinyl]-phosphat,
O,O-Dimethyl-O-[(1-methyl-2-chlor-2-diethylcarbamoyl)-vinyl]-phosphat,
O,O-Diethyl-S-(ethylthio-methyl)-phosphordithioat,
O,O-Diethyl-S-[(p-chlor-phenylthio)-methyl]-phosphordithioat,
O,O-Dimethyl-S-(2-ethylthioethyl)-phosphorthioat,
O,O-Dimethyl-S-(2-ethylthioethyl)-phosphordithioat,
O,O-Dimethyl-S-(2-ethylsulfinyl-ethyl)-phosphorthioat,
O,O,Dimethyl-S-(2-ethylthio-ethyl)-phosphordithioat,
O,O-Diethyl-S-(2-ethylsulfinyl-ethyl)-phosphorthioat,
O,O-Diethyl-thiophosphoryliminophenyl-acetonitril,
O,O-Diethyl-S-(2-chlor-1-phtalimidoethyl)-phosphordithioat,
O,O-Diethyl-S-[6-chlor-benzoxazolon-(2)-yl(3)]-methyldithiophosphat,
O,O-Dimethyl-S-[2-methoxy-1,3,4-thiadiazol-5-[4H]-onyl-(4)-methyl]-phosphordithioat,
O,O-Dimethyl-O-[3,5,6-trichlor-pyridyl-(2)]-phosphorthioat,
O,O-Diethyl-O-(2-pyrazinyl)-phosphorthioat,
O,O-Diethyl-O[2-isopropyl-4-methyl-pyrimidinyl(6)]-phosphorthioat,
O,O-Diethyl-O-[2-(diethylamino)-6-methyl-4-pyrimidinyl]-thionophosphat,
O,O-Dimethyl-S-(4-oxo-1,2,3-benzotriazin-3-[4H]-yl-methyl)-phosphordithioat,
O,O-Dimethyl-S-[(4,6-diamino-1,3,5-triazin-2-yl)-methyl]-phosphordithioat,
O,O-Diethyl-(1-phenyl-1,2,4-triazol-3-yl)-thionophosphat,
O,S-Dimethyl-phosphor-amido-thioat,
O,S-Dimethyl-N-acetyl-phosphoramidothioat,
$\gamma$-Hexachlorcyclohexan,
1,1-Di-(p-methoxyphenyl)-2,2,2-trichlor-ethan,
6,7,8,9,10,10-Hexachloro-1,5,5a,6,9,9a-hexahydro-6,9-methano-2,4,3-benzodioxa-thiepin-3-oxid,
Pyrethrine,
DL-2-Allyl-3-methyl-cyclopenten-(2)-on-(1)-yl-(4)-DL-cis,-trans-chrysanthemat,
5-Benzyl-furyl-(3)-methyl-DL-cis,-trans-chrysanthemat,
3-Phenoxybenzyl($\pm$)-cis,trans-2,2-di-methyl-3-(2,2-dichlorvinyl)-cyclopropancarboxylat,
$\alpha$-Cyano-3-phenoxybenzyl($\pm$)-cis,trans-2,2-dimethyl-3-(2,2-dichlorvinyl)-cyclopropancarboxylat,
(s)-$\alpha$-Cyano-3-phenoxybenzyl-cis(1R,3R)-2,2-dimethyl-3-(2,2-dibromvinyl)-cyclopropancarboxylat,
3,4,5,6-Tetrahydrophtalimidoethyl-DL-cis,trans-chrysanthemat,
2-Methyl-5-(2-propinyl)-3-furylmethyl-chrysanthemat,
($\alpha$-Cyano-3-phenoxybenzyl)-$\alpha$-isopropyl-4-chlorphenylacetat.

Die folgenden Beispiele belegen die biologische Wirkung der neuen Verbindungen. Vergleichsmittel ist der bekannte Wirkstoff N-(2-Methylbenzoyl)-N'-[3-chlor-4-(4-chlorphenoxy)-phenyl]-harnstoff(DE-OS 25 31 202).

Die Numerierung der Wirkstoffe entspricht der der tabellarischen Auflistung.

Beispiel 1

Zuchtversuch mit Moskito-Larven (Aedes aegypti)

200 ml Leitungswasser werden mit der Wirkstoffaufbereitung, die 10 Gew.-% Wirkstoff und 90 Gew.-% eines Emulgatorgemisches aus 10 Gew.-% ethoxyliertem Rizinusöl, 20 Gew.-% ethoxyliertem Isooctylphenol und 70 Gew.-% Cyclohexanon enthält, versetzt und darauf mit 30 bis 40 Aedes-Larven im 4. Stadium belegt.

Die Versuchstemperatur beträgt 25° C. Beurteilt werden Verpuppung und Schlüpfen der Imagines, wobei eine unbehandelte Kontrolle als Maßstab dient. Während der Versuchsdauer von 10 bis 12 Tagen wird einmal mit einem herkömmlichen pulverrisierten Fischfutter gefüttert.

In diesem Test zeigen die Wirkstoffe Nr. 1, 2, 3, 4, 9, 14, 15, 16, 18, 21, 22, 24, 25, 26, 28, 52 und 54 eine bessere Wirkung als das Vergleichsmittel.

Beispiel 2

Zuchtversuch mit Stubenfliegen (Musca domestica)

50 g eines Nährbodens aus

100 Teilen Wasser
10 Teilen Bäckerhefe
10 Teilen Trockenmilch
1 Teil Agar

werden in warmem Zustand mit der wäßrigen Aufbereitung einer Mischung aus 10 Gew.-% des Wirkstoffes und 90 Gew.-% einer Mischung aus Netzmittel und inertem Trägermaterial gründlich durchmischt.

Nach dem Erkalten belegt man den Nährboden mit ca. 0,1 ml Fliegeneiern und beobachtet deren Entwicklung über eine Woche. Die Versuchstemperatur liegt bei 20° C.

In diesem Test zeigen die Wirkstoffe Nr. 15, 18, 28, 54 eine sehr gute Wirkung, während das Vergleichsmittel unwirksam ist.

Beispiel 3

Zuchtversuch mit Mittelmeerfruchtfliegen (Ceratitis capitata)

Die Versuche werden in 100 ml-Plastik-Bechern, die mit 40 g eines Nährbodens aus Karottenpulver und Wasser (1 : 3) mit Hefezusatz gefüllt sind, durchgeführt. Der Wirkstoff wird als wäßrige Aufbereitung in den fertigen Brei eingerührt, und dieser wird mit 100 bis 200 frischen Eiern beimpft. Die Becher werden bei 24 bis 26° C geschlossen aufbewahrt; nach ca. einer Woche kann die Entwicklung beurteilt werden.

In diesem Test zeigen die Wirkstoffe Nr. 2, 4, 28 eine stärkere Wirkung als das Vergleichsmittel.

Beispiel 4

Zuchtversuch mit Reismehlkäfern (Tribolium castaneum)

10 g Weizenmehl, die mit 0,02 bzw. 0,5 mg Wirkstoff innig vermischt sind, werden in 250 ml Plastikflaschen gefüllt und mit 20 Reismehlkäfern belegt. Nach der Eiablage (nach 14 Tagen) siebt man die Käfer ab. Das Mehl wird in die Flaschen zurückgefüllt und lagert bis zum Schlüpfen der nächsten Käfergeneration bei + 24° C.

In diesem Test zeigen die Wirkstoffe Nr. 2, 4, 9 eine bessere Wirkung als das Vergleichsmittel.

Beispiel 5

Zuchtversuch mit Baumwollwanzen (Dysdercus intermedius)

50 g Baumwollsaat werden für 24 Stunden in der wäßrigen Wirkstoffaufbereitung von 10 Gew.-% Wirkstoff und 90 Gew.-% eines Emulgatorgemisches aus 10 Gew.-% ethyoxyliertem Rizinusöl, 20 Gew.-% ethoxyliertem Isooctylphenol und 70 Gew.-% Cyclohexanon eingequollen. Die überstehende Flüssigkeit wird abgeschüttet.

Darauf bringt man 20 Wanzen im vorletzten Larvenstadium in 1 l-Gläsern, deren Boden mit feuchtem Sand bedeckt ist. Diese Tiere erhalten 7 Tage lang die vorbehandelten Samen als ausschließliche Nahrung. Anschließend wird unbehandeltes Futter geboten.

Es wird beobachtet, ob die Tiere

a) überleben und
b) sich zu Adulten häuten.

In diesem Test zeigt der Wirkstoff Nr. 28 eine bessere Wirkung als die Vergleichsmittel.

Beispiel 6

Zuchtversuch mit Larven der Mehlmotte (Ephestia kuehniella)

Weizenmehl, welches stark mit Eiern der Mehlmotte belegt ist, wird mit den Wirkstoffen innig gemischt. 10 g dieses Mehls werden dann in 250 ml Glasflaschen abgefüllt und bei 22° C gelagert. Nach 4 Wochen beurteilt man die Entwicklung der Larven.

In diesem Test zeigen die Wirkstoffe Nr. 2, 4, 9, 28 eine starke Wirkung.

**Patentansprüche**

1. N-Benzoyl-N'-phenoxylphenylharnstoffe der Formel

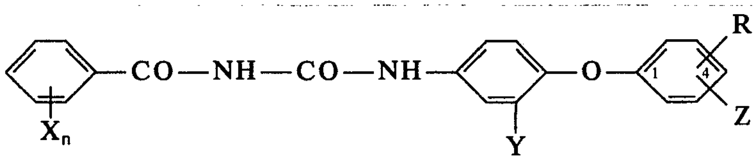

(I)

in der

Z Chlor in 3-Stellung oder Methyl,
R Fluor, Chlor, Brom, einen durch Fluor, Chlor, Brom, Halogenalkyl- oder Halogenalkoxyreste mit bis zu 4 Kohlenstoffatomen gegebenenfalls substituierten Phenoxyrest, einen gegebenenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Y Wasserstoff, Fluor oder Chlor,
X Fluor, Chlor, Brom, Methyl oder Nitro und
n 2 oder 3 bedeuten, wobei die Substituenten X gleich oder verschieden sind,

in der

Z Wasserstoff bedeutet, wenn
R einen gegebenenfalls durch Fluor, Chlor, Brom, Halogenalkyl- oder Halogenalkoxyreste mit bis zu 4 Kohlenstoffatomen substituierten Phenoxyrest, einen gegebenenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Y Wasserstoff, Fluor oder Chlor,
X Fluor, Chlor, Brom, Methyl oder Nitro und
n 1, 2 oder 3 bedeuten, wobei die Substituenten X gleich oder verschieden sind,

in der

Z Wasserstoff bedeutet, wenn
R Fluor oder Brom,
Y Chlor,
X Fluor, Chlor, Brom, Methyl oder Nitro und
n 1 bedeuten,

in der

Y Brom oder Nitro bedeutet, wenn
R Fluor, Chlor, Brom, einen gegebenenfalls durch Fluor, Chlor, Brom, Halogenalkyl- oder Halogenalkoxyreste mit bis zu 4 Kohlenstoffatomen substituierten Phenoxyrest, einen gegebenenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Z Wasserstoff, Chlor in m-Stellung oder Methyl,
X Fluor, Chlor, Brom, Methyl oder Nitro und
n 1, 2 oder 3 bedeuten, wobei die Substituenten X gleich oder verschieden sind.

2. N-Benzoyl-N'-phenoxyphenylharnstoffe der Formel I gemäß Anspruch 1, dadurch gekennzeichnet, daß Z Wasserstoff, X Chlor in o-Stellung und n 1 bedeuten.

3. N-Benzoyl-N'-phenoxyphenylharnstoffe der Formel I gemäß Anspruch 1, dadurch gekennzeichnet, daß X Fluor oder Chlor in o-Stellung und n 2 bedeuten.

4. Verfahren zur Herstellung von N-Benzoyl-N'-phenoxyphenylharnstoffen der Formel I gemäß Anspruch 1, dadurch gekennzeichnet, daß man

a) eine Verbindung der Formel

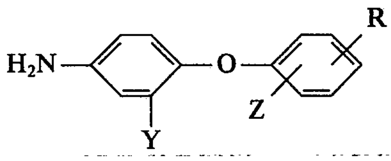

(II)

in der Y, Z und R die im Anspruch 1 genannten Bedeutungen haben, mit einem Benzoylisocyanat der Formel

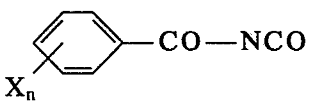

(III)

in der X und n die im Anspruch 1 genannten Bedeutungen haben, oder

b) eine Verbindung der Formel

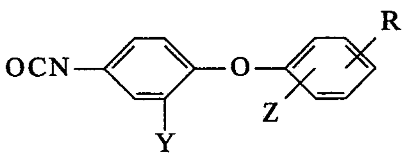

(IV)

in der Y, Z und R die im Anspruch 1 genannten Bedeutungen haben, mit einem Benzamid der Formel

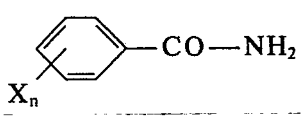

(V)

in der X und n die im Anspruch 1 genannten Bedeutungen haben, umsetzt.

5. Schädlingsbekämpfungsmittel, enthaltend inerte Zusatzstoffe und einen N-Benzoyl-N'-phenoxyphenylharnstoff der Formel

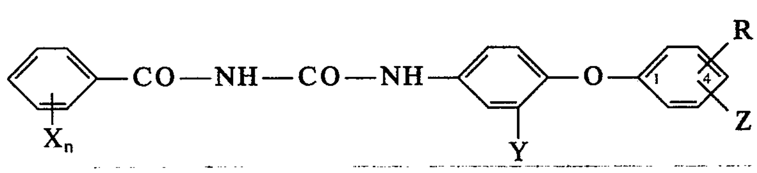

(I)

in der

Z Chlor in 3-Stellung oder Methyl,
R Fluor, Chlor, Brom, einen gegebenenfalls durch Fluor, Chlor, Brom, Halogenalkyl- oder Halogenalkoxyreste mit bis zu 4 Kohlenstoffatomen substituierten Phenoxyrest, einen gegebenenfalls durch Chlor und/oder Fluor substituierten Alkoxyrest mit 1 bis 4 Kohlenstoffatomen, einen Aklylrest mit 1 bis 3 Kohlenstoffatomen oder einen 2-Hydroxyhexahalogenisopropylrest, wobei Halogen für Fluor und/oder Chlor steht,
Y Wasserstoff, Fluor oder Chlor,
X Fluor, Chlor, Brom, Methyl oder Nitro und
n 2 oder 3 bedeuten, wobei die Substituenten X gleich oder verschieden sind,

in der

Z Wasserstoff bedeutet, wenn
R einen gegebenenfalls durch Fluor, Chlor, Brom, Halogenalkyl- oder Halogenalkoxyreste mit bis zu 4 Kohlenstoffatomen substituierten Phenoxyrest, einen gegebenenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Y Wasserstoff, Fluor oder Chlor,
X Fluor, Chlor, Brom, Methyl oder Nitro und
n 1, 2 oder 3 bedeuten, wobei die Substituenten X gleich oder verschieden sind,

in der

Z Wasserstoff bedeutet, wenn
R Fluor oder Brom,
Y Chlor und
n 1 bedeuten,

in der

Y Brom oder Nitro bedeutet, wenn
R Fluor, Chlor, Brom, einen gegebenenfalls durch Fluor, Chlor, Brom, Halogenalkyl- oder Halogenalkoxyreste mit bis zu 4 Kohlenstoffatomen substituierten Phenoxyrest, einen gegebenfalls durch Chlor und/oder Fluor substituierten Alkoxyrest mit 1 bis 4 Kohlenstoffatomen, einen Alkylrest mit 1 bis 3 Kohlenstoffatomen oder einen 2-Hydroxyhexahalogenisopropylrest, wobei Halogen für Fluor und/oder Chlor steht,
Z Wasserstoff, Chlor in m-Stellung oder Methyl-
X Fluor, Chlor, Brom, Methyl oder Nitro und
n 1, 2 oder 3 bedeuten, wobei die Substituenten X gleich oder verschieden sind,

als Wirkstoff.

6. Schädlingsbekämpfungsmittel, enthaltend inerte Zusatzstoffe und einen N-Benzoyl-N'-phenoxyphenylharnstoff der Formel I gemäß Anspruch 1, in der Z Wasserstoff, X Chlor in o-Stellung und n 1 bedeuten.

7. Schädlingsbekämpfungsmittel, enthaltend inerte Zusatzstoffe und einen N-Benzoyl-N'-phenoxyphenylharnstoff der Formel I gemäß Anspruch 1, in der X Fluor oder Chlor in o-Stellung und n 2 bedeuten.

**Claims**

1. An N-benzoyl-N'-phenoxyphenylurea of the formula

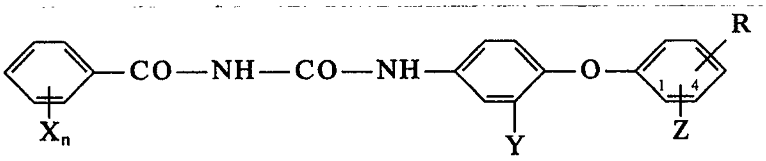

(I)

where

Z is chlorine in the 3-position or methyl,

R is fluorine, chlorine, bromine, phenoxy which is unsubstituted or substituted by fluorine, chlorine, bromine, or haloalkyl or haloalkoxy of up to 4 carbon atoms, alkoxy of 1 to 4 carbon atoms which is unsubstituted or substituted by chlorine and/or fluorine, alkyl of 1 to 3 carbon atoms, or 2-hydroxyhexahaloisopropyl, halogen being fluorine and/or chlorine,
Y is hydrogen, fluorine or chlorine,
X is fluorine, chlorine, bromine, methyl or nitro, the substituents X being identical or different, and
n is 2 or 3,

or where

Z can also be hydrogen when
R is phenoxy which is unsubstituted or substituted by fluorine, chlorine, bromine, or haloalkyl or haloalkoxy of up to 4 carbon atoms, alkoxy of 1 to 4 carbon atoms which is unsubstituted or substituted by chlorine and/or fluorine, alkyl of 1 to 3 carbon atoms, or 2-hydroxyhexahaloisopropyl, halogen being fluorine and/or chlorine,
Y is hydrogen, fluorine or chlorine,
X is fluorine, chlorine, bromine, methyl or nitro, the substituents X being identical or different, and
n is 1, 2 or 3,

or where

Z can also be hydrogen when
R is fluorine or bromine,
Y is chlorine,
X is fluorine, chlorine, bromine, methyl or nitro, and
n is 1,

or where

Y can also be bromine or nitro when
R is fluorine, chlorine, bromine, phenoxy which is unsubstituted or substituted by fluorine, chlorine, bromine, or haloalkyl or haloalkoxy of up to 4 carbon atoms, alkoxy of 1 to 4 carbon atoms which is unsubstituted or substituted by chlorine and/or fluorine, alkyl of 1 to 3 carbon atoms, or 2-hydroxyhexahaloisopropyl, halogen being fluorine and/or chlorine,
Z is hydrogen, chlorine in the m-position or methyl,
X is fluorine, chlorine, bromine, methyl or nitro, the substituents X being identical or different, and
n is 1, 2 or 3.

2. An N-benzoyl-N′-phenoxyphenylurea of the formula I as claimed in claim 1, wherein Z is hydrogen, X is chlorine in the o-position, and n is 1.

3. An N-benzoyl-N′-phenoxyphenylurea of the formula 1 as claimed in claim 1, wherein X is fluorine or chlorine in the o-position, and n is 2.

4. A process for the preparation of an N-benzoyl-N′-phenoxyphenylurea of the formula 1 as claimed in claim 1, wherein

a) a compound of the formula

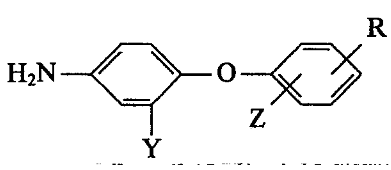

(II)

where Y, Z and R have the meanings given in claim 1, is reacted with a benzoyl isocyanate of the formula

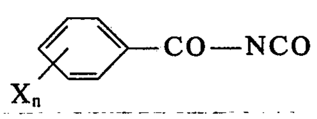

(III)

where X and n have the meanings given in claim 1, or

b) a compound of the formula

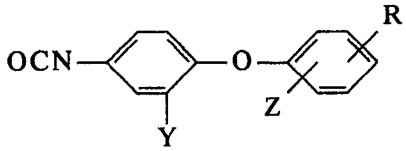

(IV)

where Y, Z and R have the meanings given in claim 1, is reacted with a benzamide of the formula

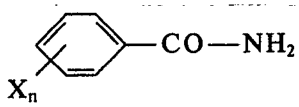

(V)

where X and n have the meanings given in claim 1.

5. A pesticide containing inert additives and, as active ingredient, an N-benzoyl-N′-phenoxyphenylurea of the formula

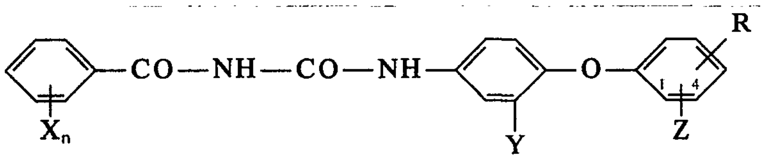

(I)

where

Z is chlorine in the 3-position or methyl,
R is fluorine, chlorine, bromine, phenoxy which is unsubstituted or substituted by fluorine, chlorine, bromine, or haloalkyl or haloalkoxy of up to 4 carbon atoms, alkoxy of 1 to 4 carbon atoms which is unsubstituted or substituted by chlorine and/or fluorine, alkyl of 1 to 3 carbon atoms, or 2-hydroxyhexahaloisopropyl, halogen being fluorine and/or chlorine,
Y is hydrogen, fluorine or chlorine,
X is fluorine, chlorine, bromine, methyl or nitro, the substituents X being identical or different, and
n is 2 or 3,

or where

Z can also be hydrogen when
R is phenoxy which is unsubstituted or substituted by fluorine, chlorine, bromine, or haloalkyl or haloalkoxy of up to 4 carbon atoms, alkoxy of 1 to 4 carbon atoms which is unsubstituted or substituted by chlorine and/or fluorine, alkyl of 1 to 3 carbon atoms, or 2-hydroxyhexahaloisopropyl, halogen being fluorine and/or chlorine,
Y is hydrogen, fluorine or chlorine,
X is fluorine, chlorine, bromine, methyl or nitro, the substituents X being identical or different, and
n is 1, 2 or 3,

or where

Z can also be hydrogen when
R is fluorine or bromine,
Y is chlorine, and
n is 1,

or where

Y can also be bromine or nitro when
R is fluorine, chlorine, bromine, phenoxy which is unsubstituted or substituted by fluorine, chlorine, bromine, or haloalkyl or haloalkoxy of up to 4 carbon atoms, alkoxy of 1 to 4 carbon atoms which is unsubstituted or substituted by chlorine and/or fluorine, alkyl of 1 to 3 carbon atoms, or 2-hydroxyhexahaloisopropyl, halogen being fluorine and/or chlorine,
Z is hydrogen, chlorin in the m-position or methyl,
X is fluorine, chlorine, bromine, methyl or nitro, the substituents X being identical or different, and
n is 1, 2 or 3.

6. A pesticide containing inert additives and an N-benzoyl-N'-phenoxyphenylurea of the formula I as claimed in claim 1, wherein Z is hydrogen, X is chlorine in the o-position, and n is 1.

7. A pesticide containing inert additives and an N-benzoyl-N'-phenoxyphenylurea of the formula I as claimed in claim 1, wherein X is fluorine or chlorine in the o-position, and n is 2.

## Revendications

1. N-Benzoyl-N'-phénoxyphényl-urées de la formula

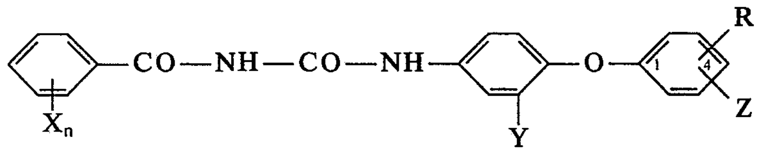

(I)

dans laquelle

Z désigne un atome de chlore en position 3 ou un radical méthyle,
R représente un atome de fluor, de chlore ou de brome, un groupe phénoxy éventuellement substitué par du fluor, du chlore ou du brome ou par des radicaux haloalkyle ou halo-alcoxy comprenant jusqu'à quatre atomes de carbone, un radical alcoxy en $C_1$ à $C_4$ éventuellement substitué par du chlore et(ou) du fluor, un radical alkyle en $C_1$ à $C_3$ ou un groupe 2-hydroxy-hexa-halo-isopropyle, l' halogène pouvant être le fluor et(ou) le chlore,
Y désigne un atome d'hydrogéne, de fluor ou de chlore,
X désigne du fluor, du chlore, du brome, un radical méthyle ou un groupe nitro et
n vaut 2 ou 3, les substituants X pouvant être edentiques ou différents;

dans laquelle

Z désigne un atome d'hydrogène, lorsque
R représente un groupe phénoxy éventuellement substitué par du fluor, du chlore ou du brome ou des radicaux halo-alkyle ou halo-alcoxy comprenant jusqu'à quatre atomes de carbone, un radical alcoxy en $C_1$ à $C_4$ éventuellement substitué par du chlore et(ou) du fluor, un radical alkyle en $C_1$ à $C_3$ ou un groupe 2-hydroxy-hexahalo-isopropyle, l'halogène pouvant être du fluor et(ou) du chlore,
Y hydrogène, fluor ou chlore,
X fluor, chlore, brome, méthyle ou nitro et
n 1, 2 ou 3, les substituants X pouvant être identiques ou différents;

dans laquelle

Z désigne un atome d'hydrogène, lorsque
R fluor ou brome,
Y chlore,
X fluor, chlore, brome, méthyle ou nitro et
n 1;

dans laquelle

Y désigne un atome de brome ou un group nitro, lorsque
R représente un atome de fluor, de chlore ou de brome, un groupe phénoxy éventuellement substitué par du fluor, du chlore ou du brome ou des radicaux haloalkyle ou halo-alcoxy comprenant jusqu'à quatre atomes de carbone, un radical alcoxy en $C_1$ à $C_4$ éventuellement substitué par du chlore et(ou) du fluor, un radical alkyle en $C_1$ à $C_3$ ou un groupe 2-hydroxy-hexahalo-isopropyle, l'halogène pouvant être du fluor et(ou) du chlore,
Z hydrogène, chlore en position méta ou méthyle,
X fluor, chlore, brome, méthyle ou nitro et
n 1, 2 ou 3, les substituants X pouvant être identiques ou différents.

2. N-Benzoyl-N'-phénoxyphényl-urées de la formule I suivant la revendication 1, caractérisées en ce que Z désigne un atome d'hydrogène et X du chlore en position ortho, n valant 1.

3. N-Benzoyl-N'-phénoxyphényl-urées de la formule I suivant la revendication 1, caractérisées en ce que X désigne du fluor ou du chlore en position ortho et n vaut 2.

4. Procédé de préparation de N-Benzoyl-N'-phénoxyphényl-urées de la formule I suivant la revendi-

cation 1, caractérisé en ce que l'on fait réagir:

a) un composé le la formule

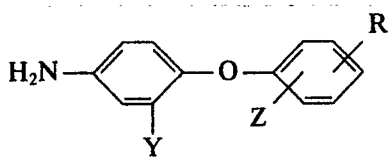

(II)

dans laquelle Y, Z et R possèdent les significations définies dans la revendication 1, avec un isocyanate de benzoyle de la formule

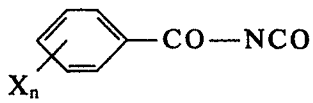

(III)

dans laquelle X et n possèdent les significations définies dans la revendication 1, ou

b) un composé de la formule

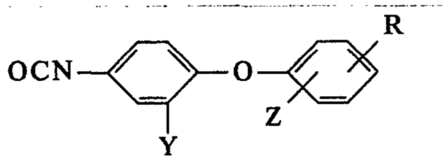

(IV)

dans laquelle Y, Z et R possèdent les significations définies dans la revendication 1, avec un benzamide de la formule

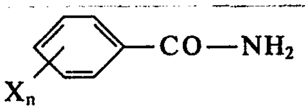

(V)

dans laquelle X et n possèdent les significations définies dans la revendication 1.

5. Composition pesticide, comprenant des additifs inertes et en tant que principe actif une N-Benzoyl-N'-phénoxyphényl-urée de la formule

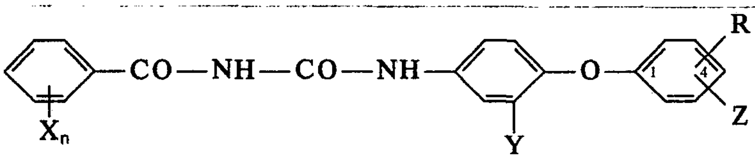

(I)

dans laquelle

- Z désigne un atome de chlore en position 3 ou un radical méthyle,
- R représente un atome de fluor, de chlore ou de brome, un groupe phénoxy éventuellement substitué par du fluor, du chlore ou du brome ou par des radicaux haloalkyle ou halo-alcoxy comprenant jusqu'à quatre atomes de carbone, un radical alcoxy en $C_1$ à $C_4$ éventuellement substitué par du chlore et(ou) du fluor, un radical alkyle en $C_1$ à $C_3$ ou un groupe 2-hydroxy-hexahalo-isopropyle, l'halogène pouvant être le fluor et(ou) le chlore,
- Y désigne atome d'hydrogène, de fluor ou de chlore,
- X désigne du fluor, du chlore, du brome, un radical méthyle ou un groupe nitro et
- n vaut 2 ou 3, les substituants X pouvant être identiques ou différents;

dans laquelle

- Z désigne un atome d'hydrogéne, lorsque
- R représente un groupe phénoxy éventuellement substitué par du fluor, du chlore ou du brome ou des radicaux halo-alkyle ou halo-alcoxy comprenant jusqu'à quatre atomes de carbone, un radical alcoxy en $C_1$ à $C_4$ éventuellement substitué par du chlore et(ou) du fluor, un radical alkyle en $C_1$ à $C_3$ ou un groupe 2-hydroxy-hexahalo-isopropyle, l'halogène pouvant être du fluor et(ou) du chlore,
- Y hydrogène, fluor ou chlore,

X fluor, chlore, brome, méthyle ou nitro et
n 1, 2 ou 3, les substituants X pouvant être identiques ou différents;

dans laquelle

Z désigne un atome d'hydrogène, lorsque
R fluor ou brome,
Y chlore et
n 1;

dans laquelle

Y désigne un atome de brome ou un groupe nitro, lorsque
R représente un atome de fluor, de chlore ou de brome, un groupe phénoxy éventuellement substitué par du fluor, du chlore ou du brome ou des radicaux haloalkyle ou halo-alcoxy comprenant jusqu'à quatre atomes de carbone, un radical alcoxy en $C_1$ à $C_4$ éventuellement substitué par du chlore et(ou) du fluor, un radical alkyle en $C_1$ à $C_3$ ou un groupe 2-hydroxy-hexahalo-isopropyle, l'halogène pouvant être du fluor et(ou) du chlore,
Z hydrogène, chlore en position méta oi méthyle,
X fluor, chlore, brome, méthyle ou nitro et
n 1, 2 ou 3, les substituants X pouvant être identiques ou différents.

6. Composition pesticide, comprenant des additifs inertes et une N-Benzoyl-N'-phénoxyphényl-urée de la formule I suivant la revendication 1, dans laquelle Z désigne de l'hydrogène et X du chlore en position ortho et n vaut 1.

7. Composition pesticide, comprenant des additifs inertes et une N-Benzoyl-N'-phénoxyphényl-urée de la formule I suivant la revendication 1, dans laquelle X désigne du fluor ou du chlore en position ortho et n vaut 2.